# EUROPEAN PATENT APPLICATION

(11) **EP 4 331 464 A1**
(43) Date of publication of application: **06.03.2024**
(21) Application number: 22795697.6
(22) Date of filing: 22.04.2022
(51) Int. Cl.: A47L 13/17, B32B 27/20, C09K 9/00, C09K 9/02, G01N 31/22, C09D 11/32, G06T 7/00, G06T 7/90, G06Q 50/10, B32B 33/00, G01N 21/78, G01N 21/80

(54) **DECONTAMINATION AGENT VISUALIZING SHEET, DECONTAMINATION AGENT VISUALIZING PARTICLE, AND DECONTAMINATION AGENT VISUALIZING METHOD USING SAME, AND INFORMATION ACQUISITION SYSTEM**

(30) Priority: 28.04.2021 JP 2021076758; 25.05.2021 JP 2021087691; 01.10.2021 JP 2021162906; 01.10.2021 JP 2021162905; 03.03.2022 JP 2022032613
(71) Applicant: CANON KABUSHIKI KAISHA, Tokyo 146-8501 (JP)
(72) Inventor: SAITO, Hiroshi, Tokyo 146-8501 (JP); SUGITA, Masaru, Tokyo 146-8501 (JP); SEKI, Masanori, Tokyo 146-8501 (JP)
(74) Representative: Canon Europe Limited
(86) International application number: PCT/JP2022/018630
(87) International publication number: WO 2022/230784

(57) **Abstract**

A disinfectant visualization sheet includes a base and a color developing portion disposed on the base, wherein the color developing portion contains a visualization agent of which color developing state changes with application of a disinfectant.

## Description

### Technical Field

The present invention relates to a disinfectant visualization sheet, a disinfectant visualization particle, and a disinfectant visualization method using the sheet or the particle.

### Background Art

In recent years, with the increasing risk of infectious diseases, cities have been locked down in consideration of the medical system. In daily life, the cases in which face-to-face communication and dialogue are not allowed have increased. In highly public spaces such as coworking spaces, an occupancy rate has reduced, productivity of users of facility with the highly public spaces has reduced, or a loss of business opportunities has also generated. There is further a fear of causing economic decline.

Under the situations described above, importance is given to work of cleaning and disinfection, and techniques for visualizing such work are disclosed. According to Patent Literature (PTL) 1, a disinfection operation in the cleaning is visualized by using a fluorescent material in an ultraviolet range. However, this technique needs a special tool and hence is not easy to implement.

PTL 2 discloses a technique of changing a developed color of a pH responsive dye by utilizing a reaction between a disinfectant and the pH responsive dye. However, the disclosed method has a difficulty in realizing long-term preservation because two reactive substances are used after mixing them into one solution.

Furthermore, PTLs 1 and 2 disclose materials capable of visualizing the completion of application of the disinfectant, but do not disclose how to record or manage application situations of the disinfectant when there are many disinfection targets. If a person records the completion of the application of the disinfectant by handwriting or by inputting data into, for example, a computer when there are many disinfection targets, the manual work needs time and effort and leads to a possibility that the application situations of the disinfectant may be erroneously recorded.

### Citation List

### Patent Literature

PTL 1: Specification of U.S. Patent No. 8519360
PTL 2: Specification of U.S. Patent Application Publication No. 2017/0336373

### Summary of Invention

### Technical Problem

An object of the present invention is to provide a disinfectant visualization sheet and a disinfectant visualization particle that can be preserved for a long term and that enable application of a disinfectant to be easily confirmed by visual check.

An object of the present invention is to provide an information acquisition system that can correctly and easily acquire information regarding the application of the disinfectant.

### Solution to Problem

According to one aspect, the present invention provides a disinfectant visualization sheet including a base and a color developing portion disposed on the base, wherein the color developing portion contains a visualization agent of which color developing state changes with application of a disinfectant.

According to another aspect, the present invention provides a disinfectant visualization particle of which color developing state changes with application of a disinfectant, the disinfectant visualization particle containing a visualization agent of which color developing state changes with the application of the disinfectant, and a carrier particle carrying the visualization agent.

According to one aspect, the present invention provides a disinfectant visualization method including a step of disposing a disinfectant visualization sheet on a to-be-disinfected surface, the disinfectant visualization sheet including a base and a color developing portion disposed on the base, the color developing portion containing a visualization agent of which color developing state changes with application of a disinfectant, and a step of applying the disinfectant to the disinfectant visualization sheet disposed on the to-be-disinfected surface.

According to another aspect, the present invention provides a disinfectant visualization method including a step of placing, on a to-be-disinfected surface, a disinfectant visualization particle of which color developing state changes with application of a disinfectant, the disinfectant visualization particle comprising a visualization agent of which color developing state changes with the application of the disinfectant and a carrier particle carrying the visualization agent, and a step of applying the disinfectant to the disinfectant visualization particle placed on the to-be-disinfected surface.

According to still another aspect, the present invention provides an information acquisition system including image information acquisition means configured to acquire image information of a to-be-disinfected region where a visualization agent of which color developing state changes with application of a disinfectant is placed, determination means configured to determine the color developing state of the to-be-disinfected region based on the image information and to output a determination result, and application information acquisition means configured to acquire information regarding the application of the disinfectant based on the determination result. Advantageous Effects of Invention

The disinfectant visualization sheet and the disinfectant visualization particle according to the present invention can be preserved for a long term and enable the application of the disinfectant to be easily confirmed by visual check.

With the information acquisition system according to the present invention, the information regarding the application of the disinfectant can be correctly and easily acquired. As a result, even if there are many disinfection targets, it is possible to realize the state in which the disinfectant is properly applied.

### Brief Description of Drawings

[Fig. 1] Fig. 1 is a schematic view of a disinfectant visualization sheet according to a first embodiment of the present invention.
[Fig. 2] Fig. 2 is a schematic view illustrating an example of the disinfectant visualization sheet according to the first embodiment of the present invention.
[Fig. 3] Fig. 3 is a schematic view of a disinfectant visualization particle according to a second embodiment of the present invention.
[Fig. 4] Fig. 4 is a schematic view illustrating another example of the disinfectant visualization sheet according to the second embodiment of the present invention.
[Fig. 5] Fig. 5 is a block diagram illustrating an information acquisition system according to an embodiment of the present invention.
[Fig. 6] Fig. 6 is a flowchart illustrating an information acquisition method according to an embodiment of the present invention.
[Fig. 7] Fig. 7 is a block diagram illustrating an example of a system according to an embodiment of the present invention.
[Fig. 8] Fig. 8 illustrates an example of a recording and management flow regarding application of the disinfectant in the embodiment of the present invention.
[Fig. 9A] Fig. 9A is a conceptual view illustrating an example of a recording and management flow regarding the application of the disinfectant in the embodiment of the present invention.
[Fig. 9B] Fig. 9B is a conceptual view illustrating the example of the recording and management flow regarding the application of the disinfectant in the embodiment of the present invention.
[Fig. 10A] Fig. 10A is a schematic view illustrating an example of a two-dimensional code in the embodiment of the present invention.
[Fig. 10B] Fig. 10B is a schematic view illustrating the example of the two-dimensional code in the embodiment of the present invention.
[Fig. 11] Fig. 11 is a schematic view illustrating an example of a terminal 502 in the embodiment of the present invention.
[Fig. 12] Fig. 12 is a schematic view illustrating an example of a server 504 in the embodiment of the present invention.
[Fig. 13] Fig. 13 illustrates Modification 1 of the recording and management flow regarding the application of the disinfectant in the embodiment of the present invention.
[Fig. 14] Fig. 14 illustrates Modification 2 of the recording and management flow regarding the application of the disinfectant in the embodiment of the present invention. Description of Embodiments

Embodiments of the present invention will be described below, but the present invention is not limited to those embodiments.

In the embodiments, a surface to be disinfected may be any surface of furniture such as a desk, a table, and a chair, a floor, a wall, and so on and may be horizontal or vertical. The to-be-disinfected surface may be a surface of, for example, wood, plastic, or metal, and can be provided by pasting a sheet to the surface or by coating a particle over the surface.

In the embodiments, a disinfectant and a visualization agent are separately prepared and are caused to react with each other in use. The visualization agent is placed on the to-be-disinfected surface, and the disinfectant is brought into contact with the to-be-disinfected surface, whereupon a color developing state (appearance) of that surface is changed. The change in the appearance can be visually recognized.

The following description is made, by way of example, in connection with a disinfectant visualization sheet and a disinfectant visualization particle.

### (First Embodiment Disinfectant Visualization Sheet)

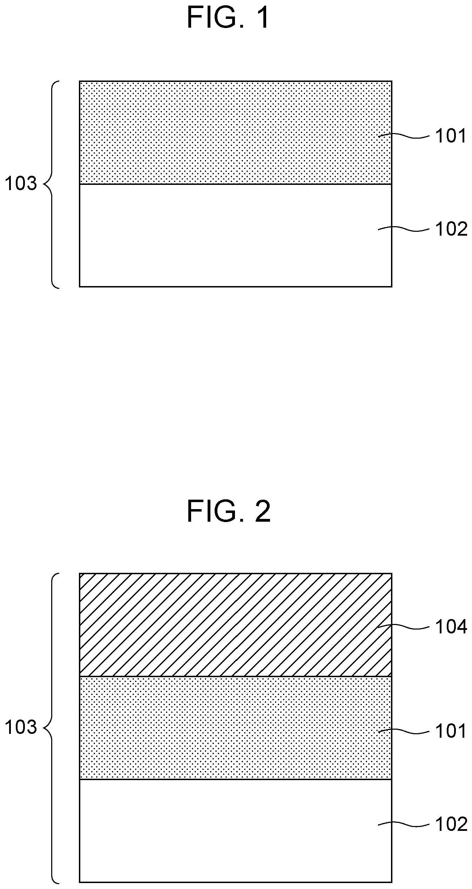

A disinfectant visualization sheet 103 according to this embodiment includes a base 102 and a color developing portion 101 disposed on the base 102 (Fig. 1). The color developing portion 101 contains the visualization agent (not illustrated) of which color developing state changes with application of the disinfectant. Using the disinfectant visualization sheet according to this embodiment is convenient because the application of the disinfectant can be confirmed by visually checking the change in the color developing state. Furthermore, the disinfectant and the visualization agent are mixed with each other at the time of applying the disinfectant to the disinfectant visualization sheet, and a time during which the disinfectant and the visualization agent are in a mixed state is short. Accordingly, materials used as the disinfectant and the visualization agent are easy to store for a long term. Here, the application of the disinfectant just needs that the disinfectant reaches the visualization agent. Thus, the application of the disinfectant may be implemented, for example, by spraying the disinfectant or by wiping a target with a duster impregnated with the disinfectant.

### <Color developing Portion>

The color developing portion in this embodiment contains at least the visualization agent. The color developing portion may contain a binder to increase adhesion to the base. Resin can be used as the binder. For example, urethane resin or polyvinyl alcohol can be used.

A thickness of the color developing portion in this embodiment is preferably 1 um or more and 80 um or less, especially preferably 5 um or more and 60 um or less, and more preferably 15 um or more and 50 um or less. By setting the thickness of the color developing portion to be 80 um or less, the adhesion to the base is increased. By setting the thickness of the color developing portion to be 1 um or more, the color developing state of the color developing portion is improved.

The color developing portion in this embodiment just needs to be disposed in at least part of the base, but it may be disposed over an entire surface of the base. The color developing portion may be disposed in at least part of the base in the shape of, for example, a polka-dotted pattern or a checkerboard pattern. When the disinfectant is applied by wiping, positions of start and end of the wiping may be each denoted by a pattern of the color developing portion to be formed. When the color developing portion is disposed on a rectangular base, a pattern of the color developing portion to be formed may be set such that a center area where the color developing portion is formed is smaller than in corners.

### <Visualization Agent>

The visualization agent used in this embodiment is not limited to a particular one as far as the color developing state of the visualization agent changes upon the reaction with the disinfectant described later. In this embodiment, the wording "change in the color developing state" indicates such a change as enabling a color difference between before and after the application of the disinfectant to be visually recognized. That change is, for example, any of a change from a visually unrecognizable state to a visually recognizable state, a change from a visually recognizable state to a visually unrecognizable state, and a visually recognizable change in color.

Here, the visually recognizable color change includes a change in shade of color and a change in color. The change in shade of color indicates, for example, a change from dark red to light red, and the change in color indicates, for example, a change from red to blue. The visually recognizable color change further includes, for example, a change from colorless to colored or vice versa.

A combination of multiple materials indicating different changes in the color developing state may be used as the visualization agent.

Chromism is known as an example of phenomenon causing the change in the color developing state. The chromism includes, for example, photochromism, thermochromism, electrochromism, acidichromism, solvatochromism, and vapochromism. In this embodiment, substances causing those phenomena are called chromic substances. More specifically, those chromic substances can be called a photochromic substance, a thermochromic substance, an electrochromic substance, an acidichromic substance, a solvatochromic substance, and a vapochromic substance.

The acidichromic substance is a substance of which color changes with a change in pH, and a color developing range is different depending on the properties of individual substances. The acidichromic substance is, for example, at least one selected from the group consisting of Metanil Yellow, Metacresol Purple, Thymol Blue, Tropaeolin O, 2,4-dinitrophenol, Methyl Yellow, Bromophenol Blue, Congo Red, Methyl Orange, Bromochlorophenol Blue, Alizarin Red S, Bromocresol Green, Methyl Orange-Xylene Cyanol FF, 2,5-dinitrophenol, Methyl Orange-Indigo Carmine, Methyl Red, Methyl Orange-Xylene Cyanol FF-phenolphthalein, Lacmoid, Chlorophenol Red, o-nitrophenol, p-nitrophenol, Bromocresol Green-Methyl Red, Bromocresol Purple, Bromophenol Red, Methyl Red-Methylene Blue, Bromothymol Blue, Neutral Red, Phenol Red, Neutral Red-Bromothymol Blue, Cresol Red, α-naphtolphthalein, Bromothymol Blue-Phenol Red, Curcumin, phenolphthalein, Cresol Red-Thymol Blue, o-cresolphthalein, α-naphtolbenzein, thymolphthalein, Thymol Blue-phenolphthalein, Alizarin Yellow GG, Alizarin Yellow R, Tropaeolin O, nitramine, 1,3,5-trinitrobenzene, Indigo Carmine, Methyl Violet, litmus, and Methyl Purple. Among the above-mentioned examples, desirable examples because of being low toxic are Metanil Yellow, Metacresol Purple, Thymol Blue, Tropaeolin O, Bromophenol Blue, Bromochlorophenol Blue, Alizarin Red S, Bromocresol Green, Methyl Red, Lacmoid, Chlorophenol Red, o-nitrophenol, Bromocresol Purple, Bromophenol Red, Bromothymol Blue, Neutral Red, Phenol Red, Cresol Red, α-naphtolphthalein, phenolphthalein, o-cresolphthalein, thymolphthalein, Alizarin Yellow GG, Alizarin Yellow R, Tropaeolin O, Methyl Violet, litmus, and Methyl Purple. More desirable examples because of causing the color change at pH of 3 or more and 11 or less are Metacresol Purple, Thymol Blue, Tropaeolin O, Bromophenol Blue, Bromochlorophenol Blue, Alizarin Red S, Bromocresol Green, Methyl Red, Lacmoid, Chlorophenol Red, o-nitrophenol, Bromocresol Purple, Bromophenol Red, Bromothymol Blue, Neutral Red, Phenol Red, Cresol Red, α-naphtolphthalein, phenolphthalein, o-cresolphthalein, thymolphthalein, Alizarin Yellow GG, Alizarin Yellow R, litmus, and Methyl Purple. The substance causing the color change at pH in an alkaline range is at least one selected from the group consisting of p-nitrophenol, Bromothymol Blue, Neutral Red, Phenol Red, Cresol Red, phenolphthalein, cresolphthalein, thymolphthalein, Alizarin Yellow, Tropaeolin, and Indigo Carmine. It is desirable that the color change should occur in an alkaline range when the disinfectant used is alkaline, and in an acidic range when the disinfectant used is acidic.

Two or more types of visualization agents may be used. Using, for example, visualization agents keeping developed colors for different times is desirable in that the visualization agents can serve as indicators for indicating the time lapsed after contact with the disinfectant.

The visualization agent may be placed to indicate character information, image information (figure), or both. The character information and the image information are not limited to specific types. However, it deems that, if expression of the character or/and image information appears in a fashion of delighting users using the disinfected regions, the users are more likely to voluntarily apply the disinfectant. The color of the visualization agent changes with vaporization of the disinfectant to restore the original one. Here, the word "restore" indicates that the color of the visualization agent returns to the state before the application of the disinfectant. This can also be said that the color developing state is reversible. In other words, the visualization agent used in this embodiment may have the color developing state that changes for a certain time with the application of the disinfectant and then returns to the state before the application of the disinfectant. For example, the color developing state may change to the visually recognizable state with the application of the disinfectant to the visualization agent that is in the visually unrecognizable state, and may return to the visually unrecognizable state after the lapse of a certain time. When the visualization agent is reversible as described above, the visualization agent can be used in a situation in which the disinfection needs to be performed repeatedly.

Two or more types of visualization agents may be used. Using, for example, visualization agents keeping developed colors for different times is desirable in that the visualization agents can serve as indicators for indicating the time lapsed after the contact with the disinfectant.

### <Base>

The base used in this embodiment may be any type of member as far as the color developing portion can be disposed on the base.

The base is preferably a resin sheet because the resin sheet is light and flexible. Materials of the resin sheet include, for example, polyester resin such as polyethylene terephthalate, polybutylene terephthalate, or a polyethylene terephthalate/isophthalate copolymer; polyolefin resin such as polyethylene, polypropylene, or polymethylpentene; polyfluoroethylene resin such as polyvinyl fluoride, polyvinylidene fluoride, polytetrafluoroethylene, or an ethylene-tetrafluoroethylene copolymer; aliphatic polyamide resin such as nylon 6 or nylon 6,6; vinyl polymer resin such as polyvinyl chloride, a vinyl chloride/vinyl acetate copolymer, an ethylene/vinyl acetate copolymer, an ethylene/vinyl alcohol copolymer, polyvinyl alcohol, or vinylon; cellulose resin such as cellulose triacetate or cellophane; acrylic resin such as polymethyl methacrylate, polyethyl methacrylate, polyethyl acrylate, or polybutyl acrylate; and other synthetic resins such as polystyrene, polycarbonate, polyarylate, and polyimide. The resin sheet may be used as one type of sheet, or a composite or laminated sheet formed by two or more types of sheets. The base preferably includes an accepting layer to retain the color developing portion. This is because, when the visualization agent is ink, the accepting layer can easily retain the ink.

The accepting layer of the base in this embodiment is desired not to react with the visualization agent. For instance, when the visualization agent develops the color in the alkaline range, the accepting layer is desirably neutral or acidic. If the accepting layer and the visualization agent react with each other, this is undesirable because the visualization agent develops the color before reacting with the disinfectant.

The base in this embodiment may be transparent, opaque, or colored. When the visualization agent has a color before reacting with the disinfectant, the base is desirably colored in a color close to that of the visualization agent.

Release paper, a metal plate, a piece of wood, or the like can be used as the base in this embodiment. The base may further include a bonding portion for bonding of the base to another member. The bonding portion may be disposed on the entirety or part of a surface of the base. When the bonding portion is disposed on part of the base surface, the base is easily peelable. The base is also easily peelable when the bonding portion contains UV curable resin.

When the base is made of paper, the color developing portion may contain, for example, an anionic, cationic, or nonionic surfactant to make the visualization agent more compatible with the paper. The nonionic surfactant, such as polyoxyethylene alkyl ether, polyoxyethylene aliphatic ester, polyoxyethylene alkyl phenyl ether, a polyoxyethylene-polyoxypropylene block copolymer, or an acetylene glycol compound, is preferably used as the surfactant.

### <Retaining Portion>

The disinfectant visualization sheet 103 according to this embodiment may include a retaining portion 104 to retain the disinfectant on the color developing portion 101 (Fig. 2). Since the retaining portion 103 enables the disinfectant to be retained for a longer time, it is possible to prolong a time during which the disinfectant is reacting with the visualization agent and developing the color. Furthermore, the color developing time can be controlled by appropriately selecting a material used as the retaining portion 103. For example, a time taken to change from the visually recognizable color-developed state to the visually unrecognizable state can be controlled.

Because of having the above-described function, the retaining portion can also be said as a color-disappearing time control portion.

The retaining portion desirably includes voids to retain the applied disinfectant. The retaining portion may include, for example, multiple inorganic particles with voids formed between the inorganic particles. The inorganic particles used here are at least one selected from the group consisting of pearl-necklace silica particles, chain silica particles, spherical colloidal silica, aspherical colloidal silica, alumina particles, titania particles, and zirconia particles. A peak position of particle size distribution (hereinafter simply called a "particle size") measured for particle sizes of the inorganic particles in accordance with the dynamic light scattering method is present preferably at 1 nm or more and 300 nm or less, more preferably at 1 nm or more and 150 nm or less, and especially preferably at 1 nm or more and 60 nm or less. Particularly, the particle size of the pearl-necklace silica particles is preferably 20 nm or more and 50 nm or less. The particle size of the chain silica particles is desirably 1 nm or more and 10 nm or less. By setting the particle size to be 300 nm or less, an amount of the voids can be sufficiently ensured. By setting the particle size to be 1 nm or more, it is possible to sufficiently increase pore diameters of the voids formed by the inorganic particles and to increase an absorption speed of the applied disinfectant. Regarding the pore diameters of the voids, a distribution curve of the pore diameters preferably has a maximum peak in a range of 5 nm or more and 20 nm or less. When the peak of the pore diameters is 5 nm or more, the absorption speed of the disinfectant increases. If that peak exceeds 20 nm, haze may increase in some cases. An increase of the haze results in a phenomenon that whiteness becomes conspicuous. Thus, from the viewpoint of suppressing reduction in visibility of the color developing portion as well, the above peak is desirably 20 nm or less. The pore diameters can be determined in accordance with the BET specific surface area measurement.

Resin used here can be selected from various types of resins. However, when the disinfectant is aqueous, water-soluble resin is preferably used. The water-soluble resin may be, for example, at least one selected from the group consisting of cellulose binders such as methylcellulose, methyl hydroxyethyl cellulose, methyl hydroxypropyl cellulose, and hydroxyethyl cellulose, starch and modified starch, gelatin and modified gelatin, natural polymer resins such as casein, pullulan, Arabic gum, karaya gum, and albumin, including derivatives thereof, polyvinyl alcohol and modified polyvinyl alcohol, latexes and emulsions such as SBR latex, NBR latex, a methyl methacrylate-butadiene copolymer, and an ethylene-vinyl acetate copolymer, polyacrylamide, vinyl polymers such as polyvinyl pyrrolidone, polyethyleneimine, polypropylene glycol, polyethylene glycol, and maleic anhydride, including copolymers thereof.

A thickness of the retaining portion may not need to be uniform. For example, a retention time of the disinfectant can be changed by varying the thickness in part of the retaining portion. In this case, the color developing time in the color developing portion can be changed, and the color developing portion can serve as an indicator for indicating the time lapsed after the contact with the disinfectant.

### <Protection Portion>

In this embodiment, a protection portion for protecting the color developing portion may be disposed on the color developing portion. Here, the wording "on the color developing portion" indicates not only the case in which the protection portion is disposed in contact with the color developing portion, but also the case in which the protection portion is disposed on the retaining portion when the retaining portion is disposed on the color developing portion. For instance, when the water-soluble resin is used for the retaining portion, a surface of the retaining portion is preferably coated with a protection layer because the surface becomes viscous. The protection portion in this embodiment can be made of at least one selected from the group consisting of resins such as acrylic resin, vinyl acetate resin, vinyl chloride resin, ethylene/vinyl acetate copolymer resin, polyamide resin, polyester resin, urethane resin, and polyolefin resin, and copolymer resins of the formers. Moreover, the protection portion preferably allows the disinfectant to permeate therethrough in an amount enough to cause the visualization agent to develop the color.

The protection portion in this embodiment may contain a water-resistant material to increase water resistance. The water-resistant material may be made of a binder that contains a smaller amount of hydrophilic resin, and that is highly hydrophobic and is less susceptible to hydrolysis. Practical examples of the water-resistant material include acrylic resin, polycarbonate modified urethane resin, and polyether modified urethane resin.

The above-mentioned examples may be used solely or as a combination of two or more.

The protection portion preferably contains the above-mentioned binder, which is less susceptible to hydrolysis, in such an amount that the content of water-soluble resin relative to the content of the total binder in the ink accepting layer is 20% by mass or less.

The protection portion in this embodiment may further contain a weather-resistant material to increase weather resistance. The weather-resistant material can be made of any of hindered amine compounds, including benzophenone, benzotriazole, and triazine compounds, which serve as ultraviolet absorbers and light stabilizers.

The provision of the color-disappearing time control portion is further preferable from the viewpoint of giving durability because the surface of the disinfectant visualization sheet is wiped with the disinfectant not a few times.

### <Disinfectant>

The disinfectant in this embodiment is used for the purpose of, for example, sanitization, sterilization, or disinfection, and it may be a liquid composition reacting with the visualization agent and causing the color developing state to change. Effective components of the disinfectant may be, for example, a surfactant, a basic component, an acidic component, and alcohol. Examples of the basic component may be alkali, perchloric acid, hypochlorous acid, and sodium salts of the formers. The reaction between the disinfectant and the visualization agent in this embodiment just needs to occur in a liquid or at a solid-liquid interface under normal temperature and normal pressure conditions or under conditions of heating, cooling, and/or application of pressure. For example, the above-described photochromism, thermochromism, electrochromism, acidichromism, solvatochromism, and vapochromism can be utilized as the principle of the reaction. Furthermore, the above-mentioned reaction may be irreversible or reversible. The disinfectant in this embodiment can be made of a mildly acidic material. An organic acid, such as citric acid, malic acid, lactic acid, or succinic acid, or a mildly acidic hypochlorous acid water can be used as the mildly acidic material.

### <Other Examples of Disinfectant Visualization Sheet>

In another example of the disinfectant visualization sheet according to this embodiment, the color developing portion may include visualization particles each containing the visualization agent and a carrier particle that carries the visualization agent. An aggregate of the visualization particles can function as the color developing portion. When the visualization particles are used, positions of a moisturizing layer and a layer including the visualization particles can be designed in microscopic scale, and hence the disinfectant visualization sheet can be designed with precise characteristics.

### <Invisible Portion>

In still another example of the disinfectant visualization sheet according to this embodiment, several principles can be used in a combined manner in addition to the above-described visualization agent. An invisible portion may be disposed in the above-described sheet including the color developing portion. An invisible material absorbing the light of wavelength in an ultraviolet or infrared range may be disposed in the invisible portion in this embodiment. The ultraviolet or infrared range includes a near-ultraviolet or near-infrared range. The provision of the invisible portion enables the application of the disinfectant to be further confirmed from the third person perspective in addition to the visual confirmation.

In still another example of the disinfectant visualization sheet according to this embodiment, the thermochromism can also be used in a combined manner. For example, a dye developing different colors depending on temperature is additionally mixed into the color developing portion. By using such a dye, the developed color changes depending on not only whether the presence of the disinfectant, but also a change in temperature from that in the usage environment of the disinfectant. Thus, a history of the disinfectant visualization sheet, such as a finger touch to the sheet, can be indirectly recognized. The thermochromism dye can be selected from known substances.

Among the invisible materials used in this embodiment, the dye absorbing the light of the wavelength in the infrared range can be given as at least one selected from the group consisting of a phthalocyanine dye, a naphthalocyanine dye, a metal complex dye, a polymethine dye, a quinone dye, an azo dye, a diphenylmethane or triphenylmethane dye, a radical dye, a perimidine dye, and an Au nanorod. The dye absorbing the light in the ultraviolet range and emitting fluorescence can be given as at least one selected from the group consisting of merocyanine, perylene, acridine, luciferin, pyranine, stilbene, rhodamine, coumarin, and fluorescein.

### <Visible Wavelength Range, Infrared or Ultraviolet Wavelength Range>

The visible wavelength range in this embodiment represents the light in a wavelength range of 360 nm or longer and 830 nm or shorter. The infrared wavelength range in this embodiment represents the light in a wavelength range of 900 nm or longer and 14 um or shorter, and the ultraviolet wavelength range represents the light in a wavelength range of 100 nm or longer and 400 nm or shorter.

### <Marking Composition>

The color developing portion disposed in the disinfectant visualization sheet according to this embodiment may be disposed as a marking indicating whether the application of the disinfectant (the cleaning) has been performed. A material used for the marking in this embodiment can be called a marking composition. The marking composition contains the above-described visualization agent and a solvent. The solvent may be, for example, a hydrophobic solvent or an aqueous solvent. The hydrophobic solvent may be, for example, an organic solvent such as heptane or petroleum ether, and the aqueous solvent may be, for example, water or alcohol.

The aqueous solvent used in this embodiment may be water or a mixed solvent using water as a main solvent and added with a protonic organic solvent or a non-protonic organic solvent. The organic solvent used in this embodiment is preferably mixed with or dissolved in water at any desired ratio and is preferably a homogeneously mixed solvent containing 50% by mass or more of water. Deionized water (ion-exchange water) or ultrapure water is preferably used as the water.

The protonic organic solvent is an organic solvent with a hydrogen atom bonded to oxygen or nitrogen (namely, an acidic hydrogen atom). The non-protonic organic solvent is an organic solvent with no acidic hydrogen atom. Examples of the organic solvent may be alcohols, alkylene glycols, polyalkylene glycols, glycol ethers, glycol ether esters, carboxylic amides, ketones, keto-alcohols, cyclic ethers, and so on.

For example, water, a water/ethanol mixed solvent, a water/ethylene glycol mixed solvent, or a water/N-methyl pyrrolidone mixed solvent can be preferably used as the aqueous solvent. The content of the water is preferably 10.0% by mass or more and 90.0% by mass or less and more preferably 50.0% by mass or more and 90.0% by mass or less on the basis of the total mass of the composition.

The content of the water-soluble organic solvent in the composition is preferably 5.0% by mass or more and 90.0% by mass or less and more preferably 10.0% by mass or more and 50.0% by mass or less on the basis of the total mass of the composition.

### <Other Additives>

The marking composition used in this embodiment may optionally contain, as required, polyalcohols such as trimethylolpropane and trimethylolethane, urine, and other water-soluble organic compounds including urine derivatives such as ethylene urine, in addition to the above-mentioned components. The marking composition used in this embodiment may further optionally contain, as required, various additives such as a surfactant, a pH regulator, an anticorrosive agent, an antiseptic agent, a fungicide, an oxidation inhibitor, a reduction inhibitor, a vaporization accelerator, a chelating agent, and water-soluble resin. Examples of the surfactant may be anionic, cationic, and nonionic surfactants. The content of the surfactant in the marking composition is preferably 0.1% by mass or more and 5.0% by mass or less and more preferably 0.1% by mass or more and 2.0% by mass or less on the basis of the total mass of the composition. Nonionic surfactants, such as polyoxyethylene alkyl ether, polyoxyethylene aliphatic ester, polyoxyethylene alkyl phenyl ether, a polyoxyethylene-polyoxypropylene block copolymer, and acetylene glycol compounds, are preferable examples of the surfactant in practical use.

When an image is recorded on a recording medium by discharging the marking composition in this embodiment from an inkjet recording head, the marking composition is preferably used under proper control of surface tension and viscosity. More specifically, a concentration of the color developing compound in the marking composition is preferably about 5% to 20%. The surface tension of the marking composition at 25°C is preferably 10 mN/m or more and 60 mN/m or less, more preferably 20 mN/m or more and 60 mN/m or less, and particularly preferably 30 mN/m or more and 50 mN/m or less.

The viscosity of the composition at 25°C is preferably 1.0 mPa·s or more and 10 mPa·s or less and more preferably 1.0 mPa·s or more and 5 mPa·s or less.

### <Image Recording Method (Method of Applying Marking Composition)>

An image recording method used in this embodiment is concerned with a method of applying the marking composition to the base. Various methods, for example, an inkjet method, a flexographic method, a screen method, an offset method, and a spin coating method, can be optionally used as the image recording method in this embodiment. In an example, the inkjet method is a method of recording an image on the recording medium by discharging the marking composition in this embodiment from the inkjet recording head. A method of discharging the marking composition may be, for example, a method of applying dynamic energy to the marking composition or a method of applying thermal energy to the composition. A process for the inkjet recording method can be implemented by a known method except for using the marking composition in this embodiment.

In addition to the above-described image recording method, an electrophotographic method can also be used as the image recording method.

### <Toner Manufacturing Method>

A toner manufacturing method capable of being applied to the electrophotographic method in this embodiment is not limited to a particular one and can be implemented as, for example, one of the following toner manufacturing methods (1) to (3).

### (1) Pulverization Method

When toner is manufactured by a pulverization method, the visualization agent is first sufficiently mixed with binder resin, serving as a dispersion medium, and other additives by using a mixer such as a Henschel mixer or a ball mill. An obtained mixture is subjected to melt kneading with a thermal kneading machine utilizing heat and a mechanical shear force, such as a kneader or an extruder, thus bringing the resin and so on into a fully dissolved state. After cooling and solidifying a material obtained with the melt kneading, the solidified material is pulverized, and pulverized particles are classified to obtain toner particles with a desired particle size.

### (2) Suspension Polymerization Method

In a suspension polymerization method, a polymerizable monomer composition is obtained by homogeneously dispersing, for example, the visualization agent, a polymerizable monomer capable of forming the binder resin, and other additives including a polymerization initiator, a crosslinking agent, a charge regulator, and so on as required. Then, the obtained polymerizable monomer composition is dispersed and granulated in a continuous layer (for example, an aqueous phase) containing a dispersion stabilizer by using a proper stirrer. A polymerization reaction is progressed with the polymerization initiator, and the toner particles with the desired particle size are obtained.

### (3) Emulsion Aggregation Method

When toner is manufactured by an emulsion aggregation method, materials such as the visualization agent, the binder resin, and other additives are first dispersed and mixed in an aqueous medium containing a dispersion stabilizer. A surfactant may be added to the aqueous medium. Then, an aggregating agent is added to progress aggregation until resin particles with the desired toner particle size are obtained. Thereafter or at the same time as the aggregation, the resin particles are fused to each other. Shape control under heating is further performed as required, whereby toner particles are formed. Then, through a filtering and washing step and a drying step, the desired toner particles are obtained.

A step of dispersing the visualization agent into the binder resin may be performed as required during a series of toner manufacturing steps. A method of dispersing the visualization agent into the binder resin may be, for example, a method of using a master batch during the toner manufacturing steps. According to that method, the visualization agent is mixed with part of the binder resin such that the visualization agent has a high concentration. An obtained mixture is subjected to melt kneading while a high shear force is applied, thus forming the master batch in which the visualization agent is finely dispersed. Then, the master batch is further subjected to melt kneading while the master batch is diluted with the remaining binder resin. A melt kneading apparatus suitably used in forming the master batch may be, for example, a kneader, a Banbury mixer, a two-roll mill, or a three-roll mill. Those apparatuses can be used solely or in combination. A melt kneading apparatus for use in the dilution kneading of the master batch may be, for example, a two-axis kneading machine. A step of suppressing aggregation of the visualization agent during the series of toner manufacturing steps may be performed as required.

A method of suppressing the aggregation of the visualization agent during the manufacturing of the toner may be, for example, a method of performing quick cooling after a melt kneading step. In an example, a melt-kneaded material can be quickly cooled by placing the melt-kneaded material to spread in the form of a sheet on a water-cooled metal belt. The aggregation of the visualization agent caused during the cooling can be suppressed with the quick cooling. A cooling apparatus suitable for the quick cooling may be, for example, an "NR Double-Belt Cooler for High Viscosity" (made by Nippon Belting, Co., Ltd.), a "Cool-Solidifying Machine, Belt Drum Flaker" (made by NIPPON COKE & ENGINEERING, CO., LTD.), or a "Cool-Solidifying Apparatus, Drum Flaker" (made by KATSURAGI INDUSTRY, CO., LTD.).

The step of dispersing the visualization agent into the binder resin and the method of suppressing the aggregation of the visualization agent may be used in a combined manner.

### <Various Additives>

The toner may contain, as required, one or more types of additives selected from wax, a charge regulator, an external additive, and so on. Preferably, the toner in the present disclosure does not contain a coloring component for causing a fixed image to become a visible image.

### <Wax>

The wax is not limited to a particular type, but colorless or light-colored wax is preferable. Examples of such wax are as follows.

Hydrocarbon wax, ester wax, amide wax, higher aliphatic alcohol, higher aliphatic acid, and so on. Those waxes may be used solely or in combination of multiple types.

### <Charge Regulator>

The charge regulator is not limited to a particular one, but a colorless or light-colored charge regulator is preferable. Examples of such a charge regulator are as follows.

Aromatic oxycarboxylic acid, a metal compound of aromatic oxycarboxylic acid, a boron compound, quaternary ammonium salt, calixarene, resin with a sulfonic acid (salt) group, resin with a sulfonic ester group, and so on.

Those charge regulators may be used solely or in combination of multiple types.

### <External Additive>

The external additive is not limited to a particular one, but a colorless or light-colored external additive is preferable. Examples of such an external additive are as follows. Silica, alumina, titanium oxide, strontium titanate, silicon nitride, polytetrafluoroethylene, zinc stearate, and so on. A surface of the external additive may be subjected to hydrophobic treatment.

An average particle size of primary particles forming the external additive is preferably 1/10 or less of a weight average particle size (D4) of the toner particles.

### <Developer>

The toner can be used as a one-component developer, but it may be mixed with a carrier to be used as a two-component developer. A magnetic particle made of a known material, for example, a metal such as iron, ferrite, or magnetite, or an alloy of that metal and another metal, such as aluminum or lead, can be used as the carrier. The carrier may be a coated carrier that is obtained by coating a carrier surface with a coating material such as resin, or a resin dispersion-type carrier that is obtained by dispersing magnetic particles into the binder resin. A volume average particle size of the carrier is preferably 15 um or more and 100 um or less and more preferably 25 um or more and 80 um or less.

### (Second Embodiment Disinfectant Visualization Particle)

The color developing state of a disinfectant visualization particle 303 according to this embodiment changes with the application of the disinfectant. In more detail, the disinfectant visualization particle contains a visualization agent 302 of which color developing state changes with the application of the disinfectant, and a carrier particle 301 carrying the visualization agent. The disinfectant visualization particle 303 in this embodiment may include the color-disappearing time control portion. The color-disappearing time control portion is not always required to have a layer form. A porous particle can be used to form the color-disappearing time control portion. For example, at least one selected from the group consisting of a meso-porous silica particle, a porous silica particle, a porous titania particle, a porous zirconia particle, a porous ceria particle, a porous zinc oxide particle, a porous crosslinked methyl polymethacrylate particle, a porous crosslinked polystyrene particle, and a porous methyl methacrylate-styrene copolymer crosslinked particle. The disinfectant visualization particle including the color developing portion can be formed by impregnating the above-mentioned porous particle with a solution in which the visualization agent is dissolved, and then by drying them.

A surface of the porous particle may be treated to be hydrophilic or hydrophobic. When the disinfectant is aqueous, the particle surface is preferably hydrophilic. When the disinfectant is not aqueous, the particle surface is preferably hydrophobic.

The disinfectant visualization particle has high rigidity, and this is preferable in that the disinfectant visualization particle has high durability even when wiping with the disinfectant is performed not a few times.

A particle size of the disinfectant visualization particle is preferably 30 nm or more and 5 um or less, more preferably 50 nm or more and 3 um or less, and particularly preferably 80 nm or more and 1 um or less. When the particle size is set to be 30 nm or more, the particles are less likely to aggregate. When the particle size is set to be 5 um or less, the disinfectant visualization particle is less likely to precipitate when dispersed into a solution.

### <Sheet Using Disinfectant Visualization Particle>

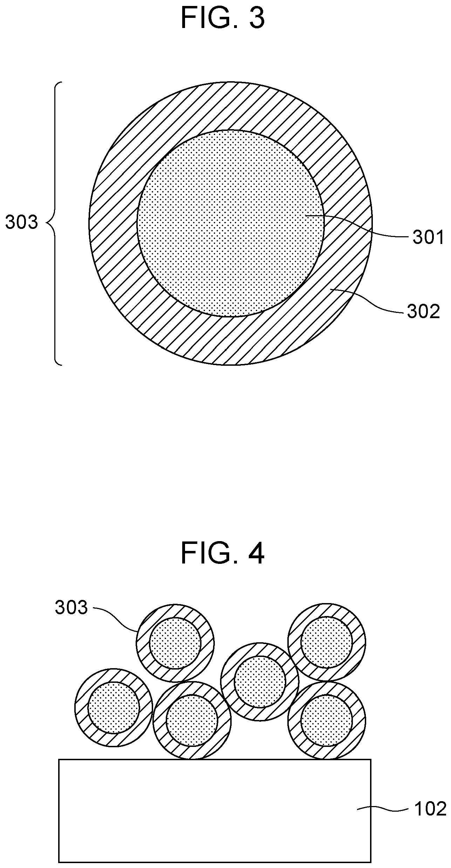

The disinfectant visualization particle according to this embodiment can be used in the form of a sheet including the disinfectant visualization particle by placing many disinfectant visualization particles 303 on the base 102 as illustrated in Fig. 4.

### <Coating Solution of Visualization Particle and Coating Layer>

The disinfectant visualization particle according to this embodiment can be used in the form of a coating solution, such as a dispersion solution or a slurry. A coating layer can also be formed by applying the coating solution to an article.

### <Variation Example of Visualization Particle>

The disinfectant visualization particle according to this embodiment is not always required to have a particulate shape. The disinfectant visualization particle may be in the form of, for example, a rod, a plate, or a film. When the disinfectant visualization particle is in the form of a film, it is particularly desirable that the color-disappearing time control portion should be disposed, from the viewpoint of maintaining the strength against the wiping with the disinfectant.

### <Third Embodiment Disinfectant Visualization Method>

An example of a disinfectant visualization method according to this embodiment includes a step of disposing the disinfectant visualization sheet according to the above-described first embodiment on the to-be-disinfected surface and a step of applying the disinfectant to the disinfectant visualization sheet disposed on the to-be-disinfected surface.

Another example of the disinfectant visualization method according to this embodiment includes a step of placing the disinfectant visualization particle according to the above-described second embodiment on the to-be-disinfected surface and a step of applying the disinfectant to the disinfectant visualization particle placed on the to-be-disinfected surface.

### (Fourth Embodiment Information Acquisition System)

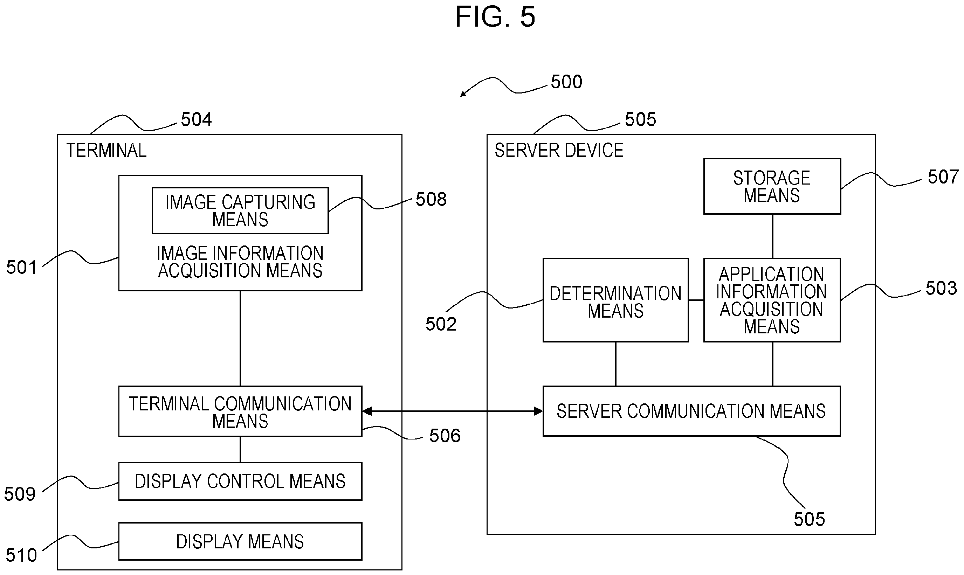

An information acquisition system 500 according to this embodiment includes at least the following components (Fig. 5) .
(i) Image information acquisition means 501 configured to acquire image information of the to-be-disinfected region where the visualization agent of which color developing state changes upon application of the disinfectant is placed.
(ii) Determination means 502 configured to determine, based on the image information, the color developing state of the to-be-disinfected region and to output a determination result.
(iii) Application information acquisition means 503 configured to acquire information regarding the application of the disinfectant based on the determination result.

The information acquisition system according to this embodiment can determine, based on the image information, whether the disinfectant has been applied to the to-be-disinfected region, and can acquire the information regarding the application of the disinfectant. Therefore, the information regarding the application of the disinfectant can be more correctly and easily acquired than when a person records the information by handwriting or by inputting data into, for example, a computer.

### <Information Regarding Application of Disinfectant>

Here, the information regarding the application of the disinfectant is, for example, at least one item of information selected from the group consisting of information regarding whether the disinfectant has been applied, time information regarding the application of the disinfectant, and information regarding a place where the disinfectant has been applied. With the acquisition of the above-mentioned multiple items of information, historical information regarding when and where the disinfectant has been applied can be acquired. The time information regarding the application of the disinfectant indicates, for example, a time at which the image information has been acquired, a time at which the determination result has been obtained, a time at which the information regarding the application of the disinfectant has been acquired, or a time lapsed from the last time at which each of the above-mentioned items of information was acquired.

The information regarding the place where the disinfectant has been applied is typically position information of the to-be-disinfected region but may be person information if the person information is linked with the position information. The place is not limited to a particular one and is, for example, a shared place utilized by a large number of unspecified people, such as a meeting room, a coworking space, a cartoon cafe, a public facility, a nursing home, a food factory, an educational organization, or a traffic organization, including chairs, desks, and toilets inside the above-described indoor places.

### <Storage Means>

The information acquisition system 500 according to this embodiment may include storage means 507 configured to store the information regarding the application of the disinfectant and the determination result. When the storage means 507 stores the information regarding the application of the disinfectant, application situations of the disinfectant can be easily managed. Thus, a configuration including the storage means 507 is particularly preferable when there are multiple regions to be disinfected. With the management of the application situations of the disinfectant, it is possible to store the information in the storage means, to refer to the stored information, and to increase efficiency of work such as the application of the disinfectant and the cleaning.

It is also possible to perform an analysis on the application situations of the disinfectant, to output a report, and to assist improvements in solution of issues.

The storage means in this embodiment may be a memory such as a ROM or a RAM, or a storage medium such as a flexible disk, a hard disk, an optical disk, an optomagnetic disk, a CD-ROM, a CD-R, a magnetic tape, a nonvolatile memory card, or a DVD.

### <Image Information Acquisition Means>

The image information acquisition means may be, for example, a camera, a video camera, a monitor camera, a terminal, a smartphone, or a tablet, each of the last three having the camera function. The image information acquisition means may acquire a still image or a moving image. A camera (wearable camera) worn on a worker who performs the disinfection operation can also be used. When the image information acquisition means acquires the moving image, it may include means (not illustrated) for acquiring an image of the to-be-disinfected region from the moving image captured during a certain period. To acquire the image of the to-be-disinfected region from the moving image, the image information acquisition means may include means for detecting a changing point in the moving image. In an example, a color (particular wavelength) in the color developing state of the visualization agent placed in the to-be-disinfected region may be detected. When the visualization agent is placed, as described later, in the form of a two-dimensional code or to indicate the character information or the image information (figure), that code or information may be detected.

In the above-described case, from the viewpoint of security, it is preferable not to store images other than the image of the to-be-disinfected region.

### <Image Capturing Means>

The image information acquisition means 501 may further include the image capturing means 508 configured to take an image of the to-be-disinfected region. The image capturing means is not limited to a particular one as far as it can capture the image of the to-be-disinfected region, especially the color developing state, in a recognizable manner.

### <Image Information>

From the viewpoint of outputting the determination result by the determination means, the visualization agent is preferably placed in the to-be-disinfected region as follows. The visualization agent is placed in the form indicating, for example, the two-dimensional code, the character information, or the image information (figure). The above-mentioned features are not limited to the case in which those features are realized with a disinfectant visualization sheet described later.

### <Display Control Means>

The information acquisition system 500 may include a terminal 504, and the terminal 504 may include the image information acquisition means 501, the determination means 502, the application information acquisition means 503, and display control means 510 configured to execute control of displaying the application information on display means 510.

### (Example of Information Acquisition System)

An example of the information acquisition system 500 according to this embodiment will be described below with reference to Fig. 5. The information acquisition system according to this embodiment includes the terminal 504 and a server device 505. The terminal 504 includes the image information acquisition means 501 and terminal communication means 506 configured to transmit the image information to the server device 505. The server device 505 includes the determination means 502, the application information acquisition means 503, and server communication means 505 configured to transmit the information regarding the application of the disinfectant to the terminal 504. The terminal communication means 506 receives the information regarding the application of the disinfectant, that information being transmitted from the server device 505. The terminal 504 may include the display control means 509 configured to execute the control of displaying the information regarding the application of the disinfectant on the display means. The terminal may further include the display means 510.

### <Terminal>

The terminal in this embodiment may be a terminal dedicated for the information acquisition system according to this embodiment or may be a universal smartphone or tablet.

### <Another Example of Information Acquisition System>

The information acquisition system may have another configuration different from that illustrated in Fig. 5. In an example, the terminal 504 may have a configuration including the image information acquisition means 501, the determination means 502, and the terminal communication means 506 configured to transmit the determination result to the server device 505. In that case, the server device 505 includes the application information acquisition means 503 and the server communication means 505 configured to transmit the information regarding the application of the disinfectant to the terminal 504. The terminal communication means 506 receives the information regarding the application of the disinfectant transmitted from the server device 505, and the terminal 504 includes the display control means 510 configured to execute the control of displaying the information regarding the application of the disinfectant on the display means 510.

A target of the image information to be acquired by the image information acquisition means may be a disinfectant visualization sheet. The disinfectant visualization sheet may include a base and a color developing portion disposed on the base, the color developing portion containing the visualization agent of which color developing state changes upon the application of the disinfectant. The disinfectant visualization sheet may be the same as the disinfectant visualization sheet according to the first embodiment.

In the disinfectant visualization sheet according to this embodiment, the visualization agent is preferably placed on the base to indicate a two-dimensional code.

### <Communication Means>

The communication means (the terminal communication means and the server communication means) in this embodiment may be a LAN (Local Area Network), and the LAN may be a wired LAN or a wireless LAN. As an alternative, the communication means may be a WAN (Wide Area Network).

### <Two-Dimensional Code>

The two-dimensional code is at least one selected from the group consisting of QR code (registered trademark), micro QR code, SP code, VeriCode, MaxiCode, CP code, Data Matrix, DataMatrix ECC200, Code1, AztecCode, INTACTA CODE, Card e, Chameleon Code, PDF417, micro PDF417, Code49, Code16K, Codablock, SuperCode, Ultra Code, RSS Composite, and AztecMesa. With the visualization agent being placed on the base to form the two-dimensional code, it is possible to read the two-dimensional code by the above-described image capturing unit and to determine, based on image information of the read two-dimensional code, that the disinfectant has been applied.

The time information regarding the application of the disinfectant and the information regarding the place where the disinfectant has been applied can be obtained from the two-dimensional code. In response to read of the two-dimensional code, character information or image information indicating the completion of the disinfection (cleaning) may be displayed on the display means in, for example, the terminal having read the two-dimensional code. When the to-be-disinfected region is in a place where a usage fee is to be paid, such as a shop or a coworking space, the terminal may be connected to a system of paying the accompanying usage fee of the shop upon the read of the two-dimensional code. In addition, a user may be motivated to perform the disinfection (cleaning) by indicating, upon the read of the two-dimensional code, information regarding grant or exchange of points available in the shop, etc. and regarding giving of a discount or a gift in the form of, for example, character information, image information, or both.

### (Information Acquisition Method)

An information acquisition method according to this embodiment includes the following steps.
(a) An image information acquisition step (S601) of acquiring image information of the to-be-disinfected region where the visualization agent of which color developing state changes upon application of the disinfectant is placed.
(b) A determination step (S602) of determining, based on the image information, the color developing state of the to-be-disinfected region and outputting a determination result.
(c) An application information acquisition step (S603) of acquiring information regarding the application of the disinfectant based on the determination result.

The information acquisition method according to this embodiment may include a display control step of executing the control of displaying the information regarding the application of the disinfectant on the display means.

An example of the information acquisition method according to this embodiment is described in more detail.

In this embodiment, the to-be-disinfected surface may be any surface of furniture such a desk, a table, or a chair, a floor, a wall, and so on and may be horizontal or vertical. The to-be-disinfected surface may be a surface of, for example, wood, plastic, or metal and can be provided by pasting a sheet to the surface or by coating a particle over the surface.

In this embodiment, the disinfectant and the visualization agent are separately prepared and are caused to react with each other in use. The visualization agent is placed on the to-be-disinfected surface, and the disinfectant is brought into contact with the to-be-disinfected surface, whereupon the color developing state (appearance) of that surface is changed. The change in the appearance can be visually recognized. The disinfectant visualization sheet according to the first embodiment and the disinfectant visualization particle according to the second embodiment can be used in the information acquisition method according to this embodiment.

### (Fifth Embodiment Disinfectant Visualization System) <System Configuration>

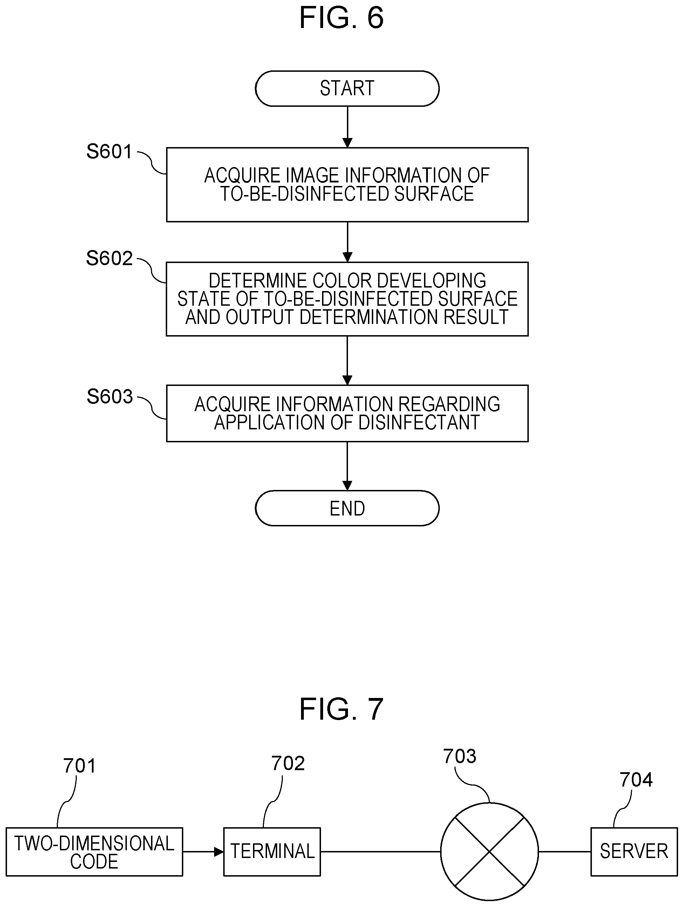

Fig. 7 illustrates a configuration of the disinfectant visualization system according to an embodiment of the present invention.

The disinfectant visualization system according to the embodiment of the present invention includes a two-dimensional code 701 formed by any of the disinfectant, the disinfectant visualization sheet, and the disinfectant visualization particle, a terminal 702, a communication network 703, and a server 704. The communication network 703 may be the Internet or an intranet and may be wireless or wired. A control unit described below is constituted by a processor, such as a CPU or an MPU, and can execute various types of control described below.

### <Recording and Management Flow>

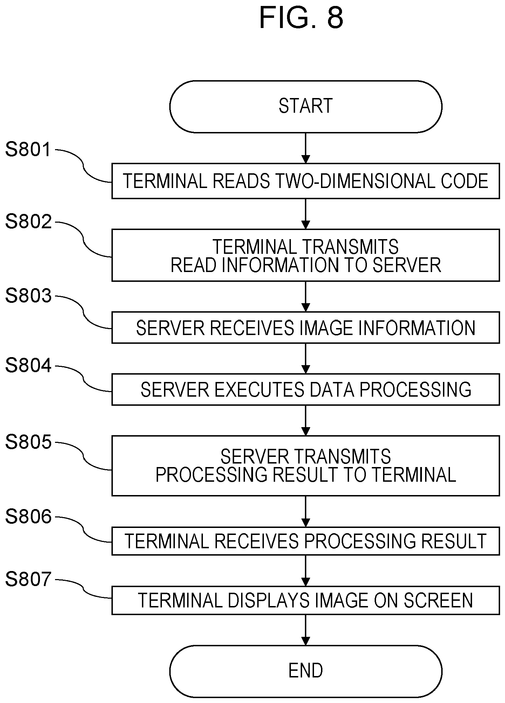

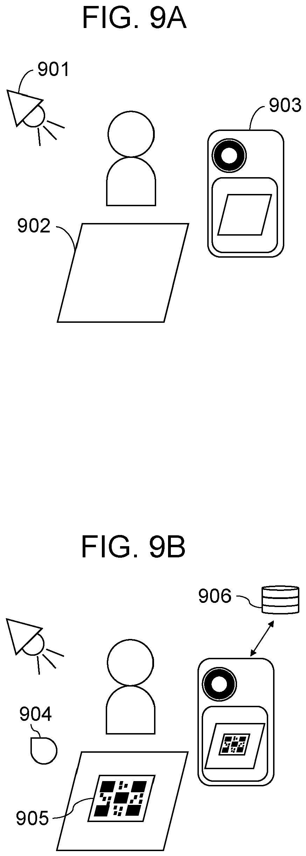

A recording and management flow in the disinfectant visualization system illustrated in Fig. 8 is described with reference to conceptual views illustrated in Figs. 9A and 9B. The system according to this embodiment mainly includes a lighting 901 in a wavelength range of 360 nm or longer and 830 nm or shorter, a disinfectant visualization sheet 902 on which a two-dimensional code 905 is formed, a terminal 903, a disinfectant 904, and a server 906. The lighting 901 may be replaced with sunlight. Fig. 9A illustrates a state before the disinfectant 904 is applied to the disinfectant visualization sheet 902, and Fig. 9B illustrates a state after the application of the disinfectant. As illustrated in Fig. 9B, the two-dimensional code 905 is visualized after the application.

In step S801, the terminal 903 reads the two-dimensional code having been visualized as illustrated in Fig. 9B. The terminal 903 extracts information to be read from the read image information. The extraction of the read information may be performed by the same device as described above or may be performed after transmitting the image information to another device.

In step S802, the terminal 903 transmits the read information to the server 906 via a communication network (not illustrated).

In step S803, the server 906 receives the read information.

In step S804, the server 906 executes data processing of the read information.

In step S805, the server 906 transmits a processing result to the terminal 903 via the communication network (not illustrated). This step can be omitted if there is no need of transmitting the processing result.

In step S906, the terminal 903 receives the processing result. This step can be omitted if there is no need of receiving the processing result.

In step S907, the terminal 903 displays the processing result on a screen.

Individual devices constituting the disinfectant visualization system will be described below.

### <Two-Dimensional Code 1001>

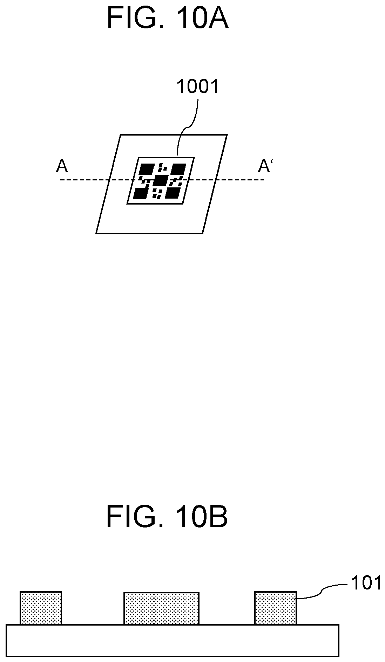

Details of the two-dimensional code 1001 are described with reference to Figs. 10A and 10B. Fig. 10B illustrates a cross-section of the two-dimensional code 1001 taken along A-A' in Fig. 10A. The two-dimensional code 1001 includes at least the color developing portion 101 in a code area of the two-dimensional code 1001. The two-dimensional code 1001 includes code at least in part thereof. The code is not always limited to a two-dimensional pattern and may be a one-dimensional pattern. As an alternative, the code may be code using color information, for example.

### <Terminal 702>

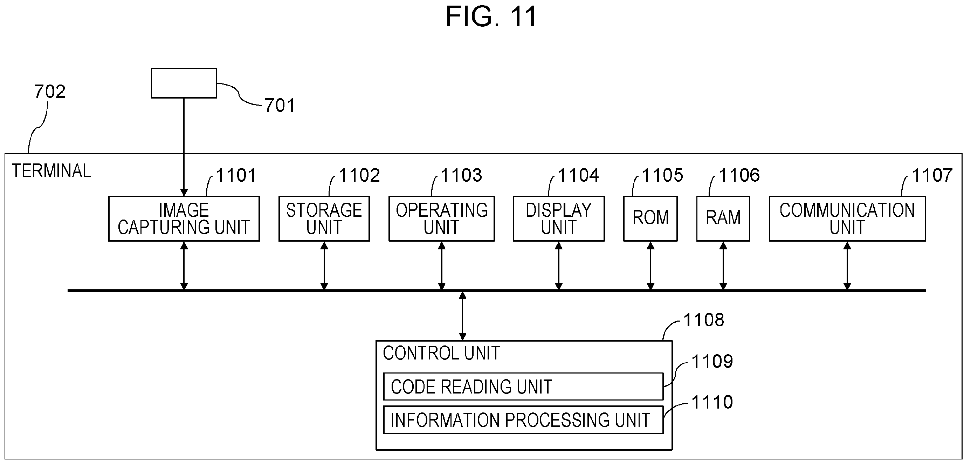

An example of the terminal 702 illustrated in Fig. 7 is described with reference to a schematic view of Fig. 11. The terminal 702 includes an image capturing unit 1101, a storage unit 1102, an operating unit 1103, a display unit 1104, a ROM 1105, a RAM 1106, a communication unit 1107, and a control unit 1108. The image capturing unit 1101 captures an image by a camera, for example, and obtains image data of the two-dimensional code 1001. The storage unit 1102 stores the image data. The ROM 1105 is implemented as a nonvolatile memory or the like and stores various programs and so on. The RAM 1006 is implemented as a volatile memory or the like and temporarily stores various kinds of information. The operating unit 1103 is implemented as a touch panel or the like. The display unit 1104 is implemented as a display or the like and displays various kinds of information to the user. The communication unit 1107 is implemented as a wireless communication module or the like and executes communication with an external device (for example, the server 704) or a communication network. However, the communication unit 1107 is not an essential component if there is no need of performing communication as in Variation Example 1 and Variation Example 2, described later, of the recording and management flow.

The control unit 1108 includes a code reading unit 1109 and an information processing unit 1110. The image data stored in the storage unit 1102 is converted to the read information by the code reading unit. While referring to a database stored in the ROM 1105 or the RAM 1106, for example, the information processing unit 1110 may add information to the read information, execute conversion of information, or store information. In an example, the information regarding the place and the time of the application of the disinfectant, the person who has applied the disinfectant, and so on can be added.

The image capturing unit 1101, the storage unit 1102, the operating unit 1103, the display unit 1104, the ROM 1105, the RAM 1106, the communication unit 1107, and the control unit 1108 are integrally constituted in Fig. 11, but they are not always required to be integral. For example, the image capturing unit 1101 may be a network camera.

### <Server 704>

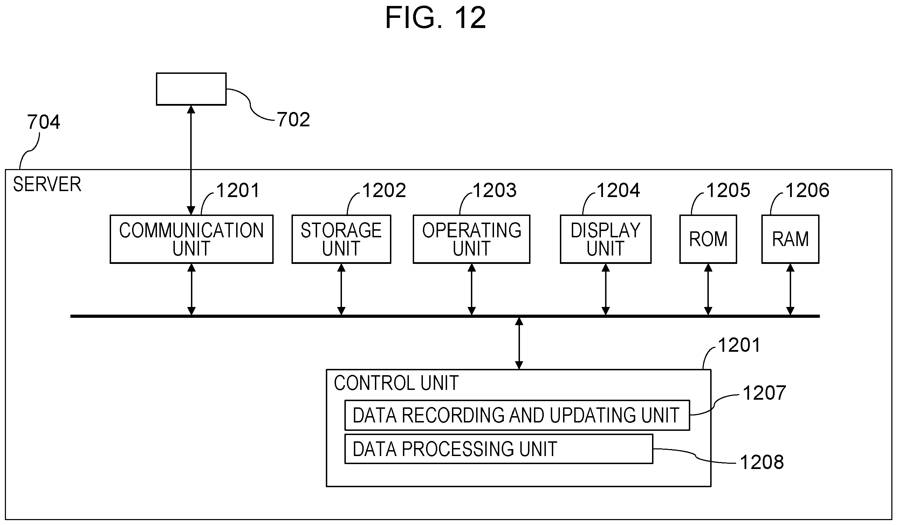

An example of the server 704 is described with reference to a schematic view of Fig. 12. The server 704 includes a communication unit 1201, a storage unit 1202, an operating unit 1203, a display unit 1204, a ROM 1205, a RAM 1206, and a control unit 1210. The communication unit 1201 is implemented as a LAN card or the like and executes communication with an external device (for example, the terminal 702) or a communication network. The ROM 1205 is implemented as a nonvolatile memory or the like and stores various programs and so on. The RAM 1206 is implemented as a volatile memory or the like and temporarily stores various kinds of information. The storage unit 1202 is implemented as an HDD (Hard Disk Drive), an SSD (Solid State Drive), a NAS (Network Attached Storage), or the like and stores various kinds of information. The operating unit 1203 is implemented as a keyboard, a mouse, or the like. The display unit 1204 is implemented as a display or the like and displays various kinds of information to the user.

The control unit 1210 includes a data recording and updating unit 1207 and a data processing unit 1208. The data recording and updating unit 1107 stores, based on the read information received by a receiving unit 1101, the application situations of the disinfectant and so on in the storage unit 1202, the ROM 1205, or the RAM 1206. The data processing unit 1208 executes data processing of information, such as addition of information to the read information or conversion of information, while referring to a database stored in the storage unit 1202, the ROM 1205, or the RAM 1206. A processing result may be communicated to the terminal 702 by using the communication unit 1201.

### <Variation Example 1 of Recording and Management Flow>

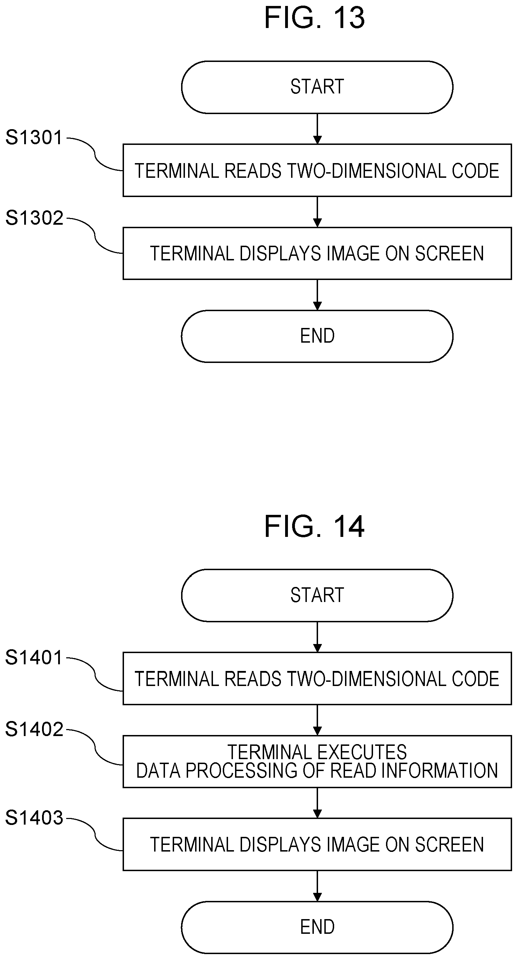

Variation Example 1 of the flow not using the server will be described below, by way of example, with reference to Fig. 13 while referring to the conceptual views illustrated in Figs. 9A and 9B as well. Variation Example 1 is suitable for use under circumferences, such as the underground, in which communication situations are poor.

Step S1301 is similar to S801.

In Step S1302, the terminal 903 displays the read information on the screen.

### <Variation Example 2 of Recording and Management Flow>

Variation Example 2 of the flow not using the server will be described below, by way of example, with reference to Fig. 14 while referring to the conceptual views illustrated in Figs. 9A and 9B as well. Variation Example 2 is suitable for use under circumferences, such as the underground, in which communication situations are poor.

Step S1401 is similar to S801.

In Step S1402, the terminal 903 executes the data processing of the read information. For example, a database for use in the data processing may be previously downloaded into the terminal 903.

In Step S1403, the terminal 903 displays a processing result on the screen.

### <Application Examples of Disinfectant Visualization System>

### [Historical Display / Cleaning Situation / Recording of Usage Information, Management]

In an example, a history, a frequency of the cleaning, a record of usage, a message, and so on may be displayed in Step S807, Step S1302, or Step S1403 depending on the application situations of the disinfectant. For example, an accurate record about the application of the disinfectant can be made in Step S804 or Step S1403. This is desirable in that the cleaning situation can be managed by utilizing the record.

Examples of the information regarding the application in Step S603 and the determination result in Step S602 are described below. A database is prepared in which code information of the two-dimensional code and position information of the two-dimensional code are linked with each other. In Step S603, the position information is acquired, from the database, as the information regarding the application by using the code information that is part of the determination result of the two-dimensional code, and the acquired position information is recorded as a cleaning history. Moreover, the time when the determination has been made, which is also part of the determination result of the two-dimensional code, may be further recorded together with the above-mentioned position information.

An example of the data processing in Step S804 or Step 1402 will be described below. The database is prepared in which the code information of the two-dimensional code and the position information of the two-dimensional code are linked with each other. The position information is acquired, from the database, as the information regarding the application by using the code information that is part of the read information obtained in Step S803 or Step S1401, and the acquired position information is recorded as the cleaning history. Moreover, the read time that is also part of the read information of the two-dimensional code may be further recorded together with the above-mentioned position information.

The above-mentioned cleaning history may be analyzed and utilized to improve the cleaning work.

The above-mentioned cleaning history may be displayed and utilized to authenticate the cleaning work.

### [Points]

In an example, points can be given in Step S804 or Step S1402, based on the above-mentioned cleaning history, to the person who has applied the disinfectant, in accordance with the record regarding the application of the disinfectant. The points can be displayed in Step S807 or Step S1403 to the person who has applied the disinfectant. An article may be given other than the points. In another example, paying or discount of a usage fee or the like may be made.

### [AR]

In an example, AR (Augmented Reality) may be displayed in superimposed relation to the two-dimensional code in Step S807, Step S1302, or Step S1403.

### [Authenticity Determination]

In an example, authenticity of the two-dimensional code may be displayed in Step S807, Step S1302, or Step S1403.

### [Time for Replacement]

In an example, the time for replacement of the two-dimensional code may be displayed in Step S807, Step S1302, or Step S1403.

### EXAMPLES

The present invention will be described in more detail below in connection with EXAMPLES and COMPARATIVE EXAMPLE, but the present invention is not limited by the following EXAMPLES as far as not departing from the gist of the present invention. It is to be noted that "part" or "%" representing an amount of a component indicates a value in terms of mass unless otherwise specified.

### <Manufacturing of Disinfectant Visualization Sheet> <EXAMPLE 1>

The visualization agent was prepared as a composition by adding ml of ultrapure water, 30 ml of ethylene glycol, and 0.1 g of a surfactant (product name; Olfine PD-005, made by Nissin Chemical Co., Ltd) to 0.5 g of phenolphthalein (made by Tokyo Chemical Industry Co., Ltd.). The composition was filled into an ink cartridge and set in an inkjet recording apparatus (PIXUS 1P7230; made by Canon Kabushiki Kaisha) of the type ejecting ink from a recording head by the action of thermal energy. In this EXAMPLE, a solid image recorded by applying eight ink droplets, each having a volume of 2.5 pL, to a unit area of 1/600 inch × 1/600 inch was defined as representing "recording duty of 100%". A solid image with the duty ratio of 100% was recorded by using the above-mentioned inkjet recording apparatus in an environment with temperature of 23°C and relative humidity of 55%. An OHP sheet (product name; CG3110, made by 3M) was used as the base. A recorded article obtained as described above was dried for 24 hours in the environment with temperature of 23°C and relative humidity of 55%, whereby a printed article (shape; rectangular image of 2 mm × 3 cm) was obtained.

The retaining portion was formed on the above-mentioned printed article as follows. 0.27 G of baric acid (made by Kishida Chemical Co., Ltd.) was added to 120 g of a dispersion liquid (Snowtex ST-PS-MO, 15 wt% of solid content, made by NISSAN CHEMICAL INDUSTRIES LTD.) containing pearl-necklace silica particles, and an obtained mixture was stirred to dissolve the baric acid. A coating solution was then prepared by adding, to the above-mentioned mixture, 40 g of a solution prepared by dissolving, as water-soluble resin, polyvinyl alcohol (Kuraray Poval 95-88, made by KURARAY CO., LTD.) with 8% of solid content into water, and stirring the mixed solution. The coating solution was coated by using a Baker applicator (made by TESTER SANGYO CO., LTD.) such that the retaining portion had a thickness h of 30 um when dried. A product after the coating was placed on a hot plate heated to 80°C for 15 minutes and was dried. As a result, Sheet 1 was obtained.

### <EXAMPLES 2 to 10>

Disinfectant visualization sheets (Sheets 2 to 10) were formed, as EXAMPLES 2 to 10, by changing the visualization agent and the thickness h of the color-disappearing time control portion as indicated in Table 1 while the other points were kept the same. The thickness h being 0 indicates that the color-disappearing time control portion is not disposed.

### [Table 1]

**Table 1**

| | Visualization Agent | Thickness h [µm] |
|---|---|---|
| EXAMPLE 1 | phenolphthalein | 30 |
| EXAMPLE 2 | phenolphthalein | 0 |
| EXAMPLE 3 | phenolphthalein | 1 |
| EXAMPLE 4 | phenolphthalein | 5 |
| EXAMPLE 5 | phenolphthalein | 20 |
| EXAMPLE 6 | o-cresolphthalein | 0 |
| EXAMPLE 7 | o-cresolphthalein | 1 |
| EXAMPLE 8 | o-cresolphthalein | 5 |
| EXAMPLE 9 | o-cresolphthalein | 20 |
| EXAMPLE 10 | o-cresolphthalein | 30 |

### <Manufacturing of Disinfectant Visualization Particle>

A visualization agent solution was prepared as follows. First, 1 g of phenolphthalein (made by Tokyo Chemical Industry Co., Ltd.) was dissolved into 90 ml of ethanol (made by Kishida Chemical Co., Ltd.), and pure water was added to obtain 100 ml of the solution. The disinfectant visualization particle was then formed by impregnating 1 g of porous titania (solid TiO₂ made by MARIMO UJIDEN Co., Ltd.) with the visualization agent solution and drying it at 80°C.

### <EXAMPLES 11 to 20>

### <Evaluation>

As the disinfectant, 0.5 wt% of sodium hypochlorite aqueous solution was used. The 1-ml disinfectant was dropped onto KimWipes, and the disinfectant was applied to the disinfectant visualization sheet by wiping the sheet with the KimWipes. The sheet was placed on white paper, and an image of the sheet was captured by a video camera. The captured moving image was converted to an image with grayscale of 0 to 255, and an absolute value of an amount of change in value between before and after the application was measured. If a change amount of the absolute value is 5 or more, the change can be visually recognized. A change amount N after one second and a retention time T during which the change amount of the absolute value was 5 or more were measured. Results are listed in Table 2.

### [Table 2]

**Table 2**

| | Sheet Name | Change Amount N After 1 Sec | Retention Time T [s] |
|---|---|---|---|
| EXAMPLE 11 | Sheet 1 | 17 | 38 |
| EXAMPLE 12 | Sheet 2 | 24 | 5 |
| EXAMPLE 13 | Sheet 3 | 23 | 11 |
| EXAMPLE 14 | Sheet 4 | 19 | 20 |
| EXAMPLE 15 | Sheet 5 | 16 | 26 |
| EXAMPLE 16 | Sheet 6 | 50 | 36 |
| EXAMPLE 17 | Sheet 7 | 46 | 40 |
| EXAMPLE 18 | Sheet 8 | 53 | 64 |
| EXAMPLE 19 | Sheet 9 | 32 | 73 |
| EXAMPLE 20 | Sheet 10 | 36 | 84 |

As seen from the change amounts N in EXAMPLES 11 to 20, the change caused by contact with the disinfectant can be sufficiently visually recognized. It is also seen from the retention times T in EXAMPLES 11 to 20 that the retention time becomes longer as the thickness of the color-disappearing time control portion increases. This is preferable in that the change can be more easily recognized.

### <EXAMPLES 21 to 30>

The read information can be acquired by printing the two-dimensional code on each of Sheets 1 to 20 and by taking a code image with a camera.

### (Other Embodiments)

The embodiments of the present invention can also be realized by a computer of a system or apparatus (for example, an application specific integrated circuit (ASIC)) that reads out and executes computer-executable instructions (for example, one or more programs) recorded on a storage medium to perform one or more of the functions of the above-described embodiments, and/or by a computer of a system or apparatus including one or more circuits for performing one or more of the functions of the above-described embodiments, and by a method performed by the computer of the system or apparatus by, for example, reading out and executing the computer-executable instructions from the storage medium to perform the one or more of the functions of the above-described embodiments and/or controlling the one or more circuits to perform the one or more of the functions of the above-described embodiments. The computer may include one or more processors (for example, central processing units (CPUs) or micro processing units (MPUs)) and may further include a network of separate computers or separate processors to read out and execute the computer-executable instructions. The computer-executable instructions may be provided to the computer from, for example, a network or the storage medium. The storage medium may include, for example, one or more of a hard disk, a random-access memory (RAM), a read only memory (ROM), a storage of distributed computing systems, an optical disk (such as a compact disc (CD), a digital versatile disc (DVD), or Blu-ray Disc (BD)?), a flash memory device, a memory card, and the like.

The present invention is not limited to the above-described embodiments and can be variously altered or modified without departing from the spirit and the scope of the present invention. The following Claims are attached herewith for the purpose of making Claims of the present invention open to the public.

This application claims the priority based on Japanese Patent Application No. 2021-076758 filed April 28, 2021, No. 2021-087691 filed May 25, 2021, No. 2021-162906 and No. 2021-162905 both filed October 1, 2021, and No. 2022-032613 filed March 3, 2022, which are hereby incorporated by reference herein in their entirety.

## Claims

1. A disinfectant visualization sheet comprising a base and a color developing portion disposed on the base,
wherein the color developing portion contains a visualization agent of which color developing state changes with application of a disinfectant and includes a retaining portion arranged to retain the disinfectant.

2. The disinfectant visualization sheet according to Claim 1, wherein the change in the color developing state indicates occurrence of any of a change from a visually unrecognizable state to a visually recognizable state, a change from a visually recognizable state to a visually unrecognizable state, and a visually recognizable change in color.

3. The disinfectant visualization sheet according to Claim 1 or 2, wherein the visualization agent contains a chromic substance.

4. The disinfectant visualization sheet according to Claim 3, wherein the chromic substance is at least one selected from the group consisting of a photochromic substance, a thermochromic substance, an electrochromic substance, a solvatochromic substance, an acidichromic substance, and a vapochromic substance.

5. The disinfectant visualization sheet according to Claim 3, wherein the chromic substance contains the acidichromic substance.

6. The disinfectant visualization sheet according to Claim 3, wherein the chromic substance contains the solvatochromic substance.

7. The disinfectant visualization sheet according to any one of Claims 1 to 6, wherein the visualization agent is at least one selected from the group consisting of Metanil Yellow, Metacresol Purple, Thymol Blue, Tropaeolin O, Bromophenol Blue, Bromochlorophenol Blue, Alizarin Red S, Bromocresol Green, Methyl Red, Lacmoid, Chlorophenol Red, o-nitrophenol, Bromocresol Purple, Bromophenol Red, Bromothymol Blue, Neutral Red, Phenol Red, Cresol Red, α-naphtolphthalein, phenolphthalein, o-cresolphthalein, thymolphthalein, Alizarin Yellow GG, Alizarin Yellow R, Tropaeolin O, Methyl Violet, litmus, and Methyl Purple.

8. The disinfectant visualization sheet according to any one of Claims 1 to 7, wherein the retaining portion includes multiple inorganic particles, and voids are formed between the inorganic particles.

9. The disinfectant visualization sheet according to Claim 8, wherein a particle size of the inorganic particles is 1 nm or more and 300 nm or less.

10. The disinfectant visualization sheet according to Claim 8 or 9, wherein the retaining portion includes at least one selected from the group consisting of pearl-necklace silica particles, spherical colloidal silica, aspherical colloidal silica, alumina particles, titania particles, and zirconia particles.

11. The disinfectant visualization sheet according to any one of Claims 1 to 10, wherein the color developing portion contains a binder.

12. The disinfectant visualization sheet according to any one of Claims 1 to 11, wherein a protection portion is disposed on the color developing portion.

13. The disinfectant visualization sheet according to any one of Claims 1 to 12, wherein an invisible material is placed on the base.

14. A disinfectant visualization particle of which color developing state changes with application of a disinfectant,
the disinfectant visualization particle comprising a visualization agent of which color developing state changes with the application of the disinfectant; and
a carrier particle carrying the visualization agent.

15. The disinfectant visualization particle according to Claim 14, wherein the change in the color developing state indicates occurrence of any of a change from a visually unrecognizable state to a visually recognizable state, a change from a visually recognizable state to a visually unrecognizable state, and a visually recognizable change in color.

16. The disinfectant visualization particle according to Claim 14 or 15, wherein the visualization agent is at least one selected from the group consisting of Metanil Yellow, Metacresol Purple, Thymol Blue, Tropaeolin O, Bromophenol Blue, Bromochlorophenol Blue, Alizarin Red S, Bromocresol Green, Methyl Red, Lacmoid, Chlorophenol Red, o-nitrophenol, Bromocresol Purple, Bromophenol Red, Bromothymol Blue, Neutral Red, Phenol Red, Cresol Red, α-naphtolphthalein, phenolphthalein, o-cresolphthalein, thymolphthalein, Alizarin Yellow GG, Alizarin Yellow R, Tropaeolin O, Methyl Violet, litmus, Methyl Purple, p-nitrophenol, Neutral Red, Phenol Red, Cresol Red, phenolphthalein, cresolphthalein, thymolphthalein, Alizarin Yellow, Tropaeolin, and Indigo Carmine.

17. The disinfectant visualization particle according to any one of Claims 14 to 16, wherein the carrier particle is at least one selected from the group consisting of a meso-porous silica particle, a porous silica particle, a porous titania particle,
a porous zirconia particle, a porous ceria particle, a porous zinc oxide particle, a porous crosslinked methyl polymethacrylate particle, a porous crosslinked polystyrene particle, and a porous methyl methacrylate-styrene copolymer crosslinked particle.

18. The disinfectant visualization particle according to any one of Claims 14 to 17, wherein a retaining portion arranged to retain the disinfectant is disposed on a surface of the disinfectant visualization particle.

19. The disinfectant visualization particle according to Claim 18, wherein the retaining portion includes at least one selected from the group consisting of pearl-necklace silica particles, spherical colloidal silica, aspherical colloidal silica, alumina particles, titania particles, and zirconia particles.

20. A disinfectant visualization sheet comprising a base and the disinfectant visualization particle according to any one of Claims 14 to 19, the disinfectant visualization particle being placed on the base.

21. A disinfectant visualization method comprising a step of disposing a disinfectant visualization sheet on a to-be-disinfected surface, the disinfectant visualization sheet comprising a base and a color developing portion disposed on the base, the color developing portion containing a visualization agent of which color developing state changes with application of a disinfectant, and a step of applying the disinfectant to the disinfectant visualization sheet disposed on the to-be-disinfected surface.

22. A disinfectant visualization method comprising a step of placing, on a to-be-disinfected surface, a disinfectant visualization particle of which color developing state changes with application of a disinfectant, the disinfectant visualization particle comprising a visualization agent of which color developing state changes with the application of the disinfectant and a carrier particle carrying the visualization agent, and a step of applying the disinfectant to the disinfectant visualization particle placed on the to-be-disinfected surface.

23. An information acquisition system comprising:
image information acquisition means configured to acquire image information of a to-be-disinfected region where a visualization agent of which color developing state changes with application of a disinfectant is placed;
determination means configured to determine the color developing state of the to-be-disinfected region based on the image information and to output a determination result; and
application information acquisition means configured to acquire information regarding the application of the disinfectant based on the determination result.
